# EUROPEAN PATENT SPECIFICATION

(11) **EP 0 734 449 B1**
(45) Date of publication and mention of the grant of the patent: **14.01.1998**
(21) Application number: 94900664.7
(22) Date of filing: 26.11.1993
(51) Int. Cl.: C12P 1/02, C09B 61/00, C12P 17/18

(54) **METHOD FOR OBTAINING FOOD COLOURING PRODUCT**
VERFAHREN ZUR GEWINNUNG VON NAHRUNGSMITTEL-FARBSTOFFEN
PROCEDE D'OBTENTION D'UN PRODUIT COLORANT ALIMENTAIRE

(43) Date of publication of application: 02.10.1996
(73) Proprietor: Kujumdzieva, Anna, 1421 Sofia (BG); Savov, Valentin, 1421 Sofia (BG); Hallet, Jean-Noel, 44300 Nantes (FR)
(72) Inventor: KUJUMDZIEVA, Anna, University St. Kliment Ohridski, Oragan Tzankov Street, 8, Sofia 1421 (BG); SAVOV, Valentin, University St. Kliment Ohridski,, Dragan Tzankov Street, 8, Sofia, 1421 (BG); DAVIDOVA, Eugenia, VNII Synthesbelok Moscow (RU); TODOROV, Tzoni, University St. Kliment Ohridski,, biology, Dragan Tzankov Street, 8, Sofia (BG); RASHEVA, Tania, University St. Kliment Ohridski,, ustrial Dragan Tzankov Street, 8, Sofia (BG)
(74) Representative: Dawidowicz, Armand
(86) International application number: BG9300016
(87) International publication number: WO9514786

(56) References cited:
- WO-A-92/13959
- FR-A- 2 353 616
- FR-A- 2 505 856
- CHEMICAL ABSTRACTS, vol. 106, no. 23, 8 June 1987, Columbus, Ohio, US; abstract no. 194726s, GRZYBOWSKI, ROMAN ET AL. 'Stimulation of the biosynthesis of pigments by monascus purpureus fungus.' page 601 ; & PRZEM. SPOZYW., vol.39, no.11, 1985, POL pages 402 - 404

## Description

### FIELD OF INVENTION

The invention reffers to a method for obtaining of a colouring agent for foodstuffs, which can find application in the food and beverage industry.

### BACKGROUND OF THE INVENTION

The fungi, belonging to genus *Monascus* are well known as producers of natural colouring agents in the food and beverage industry. They have been used in the oriental countries since centuries, mainly for colouring wine, meat and curd (1). Usually the pigments are obtained as watter or oil soluble substances stable in the pH region 2 - 10, and at high temperatures (120°C) (2).

Different methods used for cultivation of these fungi for pigment biosynthesis are known. They comprise the utilization of solid and liquid complex substrates for growth and solid phase and submerged cultivation (3, 4). Natural substrates for the fungi are different cereals and especially rice, and after that the obtained red mixture is grained and applied as powder (5). Grzybowski & Stegman have developed a process for stimulation of the biosynthesis of pigments by *Monascus purpureus* fungus using a culture medium based on milk whey, diluted 1:1 with water (6).

### TECHNICAL BACKGROUND

The aim of the invention is to create a method for obtaining of a complex colouring agent for foodstuffs.

The aim of the invention can be achieved by using strain microorganism producer of pigments, belonging to genus *Monascus.* The following strains are used: *Monascus purpureus* CBS 109.07, *Monascus purpureus* DSM 1604, *Monascus purpureus* DSM 1603, *Monascus ruber DSM* 62748, *Monascus anka* IFO 4478.

As culture medium, on which the above mentioned microorganisms can grow and produce pigments, permeate obtained from milk or whey in ratio 10 - 100 %, pH 3 - 7, is used. To this main culture medium different substances can be added: additional carbon sources 0.1 - 5%, natural starch sources 0.5 - 5 %, inorganic nitrogen sources 0.1 - 1 %, organic nitrogen sources 0.1 - 10 %, mineral salts 0.001 - 1 %. It was found that the use of whey or milk permeate with different carbon sources show synergic effect on the *Monascus* pigment production. As additional carbon sources sugars, aminosugars, sugar alcohols, alcochols and other natural sources containing starch are used. As additional nitrogen sources amonium and nitrate salts, organic nitrogen containing substances as yeast extract, malt extract, corn extract etc. can be added. Additionally mineral salts like phosphates, sulphates, sodium, magnesium and potassium salts can be used.

The cultivation methods applied in this invention are submerged - batch, fed - batch, continuous. The microorganisms from genus *Monascus* are cultivated at temperature 25 - 40°C for 2 - 5 days. The fermentation process is carried out under the following conditions: pH 4 - 8, aeration 0.3 - 1 l/l/min, agitation 200 - 500 r.p.m., pO₂ 20 - 50 %.

The fermentation broth is used without any further processing as a complex colouring agent. The culture can be applied in liquid form in foodstuffs manufacture or as freeze - dried or spray - dried product. In some cases the mycelium can be removed by filtration techniques, and when necessary the liquid product can be concentrated up to 40 - 50 % dry weight by vacuum evaporation.

The advantages of the method according to this invention can be summarized as follows: a submerged, one stage method is used, the cultivation process is short; batch and fed - batch cultivation methods are used; complex and ecologically clean product, which except pigment contains lactose, proteins, amino acids, vitamins and mineral salts is obtained; the red pigment is totally water soluble while the orange pigment is in crystaline form. As main culture medium a cheap, non - traditional substrate, a by-product from the milk processing industry, is used and through this method a very strong pollutant from the milk industry is converted into a valuable product for food and beverage industry. The method is ecologically clean, the technology is wasteless.

### EXAMPLE 1

The *Monascus purpureus* DSM 1604 strain is cultivated on solid medium containing (g/l): malt extract 5, peptone 10, glucose 20, agar 20, pH 6.0. The cultivation is carried out at 30°C for 7 days. The main fermentation is performed in a 10 l bioreactor. The strain'is cultivated on culture medium, containing ultrafiltrate of milk whey with decreased protein content. The culture medium is prepared in the following way: natural milk, cheese or yellow cheddar cheese whey are subjected to ultrafiltration on DDS equipment with 5 kDa membranes. Tne obtained permeate which contains (g/l) lactose 45 - 50, protein 0.9 - 1, nitrogen 1.0 - 1.3, phosphorus 2 - 2.5 is used as a medium for the fungus cultivation.

The cultivation of the strain producer is carried out under the following conditions: temperature 30°C, pH 6.5, air supply 0.3 l/l/min, agitation 200 r.p.m. The cultivation process has a duration of 72 h. The sugar (7), nitrogen (8), phosphorus (9), protein (10), pigment (11'), dry weight (12) contain and the proteolitic activity (13) are determined. The fermentation broth is freeze dried. In the case when the proteolitic activity is not desired a heating procedure at 120°C for 15 min is involved.

The obtained product has the following characteristics: powderlike, red, watersoluble substance, containing (mg/g dry product): lactose 970, pigment 180 - 200 OU, protein 16.8, proteolitic activity 1 U/mg protein.

### EXAMPLE 2

*Monascus purpureus* CBS 109.07 is used. The cultivation is carried out under the conditions described in EXAMPLE 1. The culture medium contains milk permeate to which the following ingredients are added (g/l): yeast extract 0.5, KH₂PO₄ 1.0, MgSO₄ 7H₂O 0.5, ZnSO₄ 7H₂O 0.001, pH 6.0. As additional carbon sources different sugars, aminosugars and alcohols are used. The results are shown on Table 1.

At the end of the cultivation process, the cultural broth is spray dried. The obtained product has the following characteristics: powderlike, red, watersoluble substance, containing pigment 250 - 550 OU / mg dry weight (Table 1).

The carbon sources added, do not influence the physical properties of the product obtained.

**Table 1.**

| Culture medium | Carbon source added 1 % | Ingradients added | Pigment Production | |
|---|---|---|---|---|
| | | | Optical Units / mg dry weight | Ratio 500/400 |
| milk permeate | - | - | 200 - 250 | 1.24 |
| | - | salts | 240 - 290 | 1.28 |
| | glucose | salts | 480 - 520 | 1.58 |
| | galactose | salts | 450 - 500 | 1.68 |
| | maltose | salts | 350 - 380 | 1.48 |
| | raffinose | salts | 380 - 420 | 1.62 |
| | sucrose | salts | 520 - 580 | 1.74 |
| | xylose | salts | 350 - 420 | 1.37 |
| | fructose | salts | 380 - 400 | 1.42 |
| | glucoseamine | salts | 320 - 350 | 1.38 |
| | galactoseamine | salts | 280 - 300 | 1.42 |
| | xylitol | salts | 280 - 300 | 1.45 |
| | manitol | salts | 290 - 320 | 1.72 |
| | ribitol | salts | 420 - 450 | 1.68 |
| | sorbitol | salts | 450 - 480 | 1.70 |
| | erythritol | salts | 360 - 400 | 1.52 |
| | meso inositol | salts | 320 - 360 | 1.44 |
| | galactitol | salts | 380 - 400 | 1.56 |
| | glycerol | salts | 440 - 480 | 1.48 |
| | ethanol | salts | 480 - 520 | 1.63 |
| | methanol | salts | 460 - 500 | 1.69 |

### EXAMPLE 3

*Monascus ruber* DSM 62748 is used. The cultivation is performed under the conditions described in EXAMPLE 2 with the exeption that as additional carbon source glucose in ratio 2 % is added. As nitrogen sources different substances are added to the culture medium (Table 2). The product obtained after spray drying of the fermentation broth has the following characteristics: powderlike, red coloured watersoluble substance with pigment content 200 - 500 OU/mg dry weight and powderlike, orange substance with pigment contain 300 - 600 OU/mg dry weight (Table 2).

**Table 2.**

| Culture medium | Nitrogen source added 0.5 % | Ingradients added | Pigment Production | |
|---|---|---|---|---|
| | | | Optical Units / mg dry weight | Ratio 500/400 |
| permeate | - | - | 200 - 250 | 1.24 |
| | - | salts | 240 - 290 | 1.28 |
| | yeast extract | salts | 420 - 460 | 1.65 |
| | malt extract | salts | 400 - 440 | 1.68 |
| | meat extract | salts | 390 - 430 | 1.54 |
| | corn extract | salts | 380 - 420 | 1.56 |
| | polypeptone | salts | 390 - 430 | 1.62 |
| | casein hydrolizate | salts | 360 - 400 | 1.58 |
| | urea | salts | 320 - 380 | 1.43 |
| | sodium-L-glutamate | salts | 340 - 400 | 1.48 |
| | malt broth | salts | 360 - 400 | 1.45 |
| | NaNO₃ | salts | 370 - 400 | 1.62 |
| | NH₄Cl | salts | 240 - 300 | 0.54 |
| | (NH₄)₂SO₄ | salts | 260 - 320 | 0.46 |

### EXAMPLE 4

*Monascus anka* IFO 4478 strain is used. The cultivation is carried out under the conditions described in EXAMPLE 1 with the exeption that the culture medium used is milk permeate 50 % with additional carbon substrates different natural starch sources in ratio 1 % (Table 3). The product obtained after spray drying has the following characteristic: powderlike, red coloured, watersoluble substance, with pigment contain 500 - 600 OU/mg dry weight (Table 3).

**Table 3.**

| Culture medium | Carbon source - natural starch substrates added 1 % | Ingradients added | Pigment production | |
|---|---|---|---|---|
| | | | Optical Units / mg dry weight | Ratio 500/400 |
| milk permeate | - | - | 200 - 250 | 1.24 |
| | - | salts | 240 - 290 | 1.28 |
| | wheat flour | salts | 540 - 580 | 1.74 |
| | rice flour | salts | 560 - 600 | 1.78 |
| | corn flour | salts | 580 - 640 | 1.88 |
| | cotton flour | salts | 530 - 570 | 1.65 |
| | wheat starch | salts | 520 - 560 | 1.60 |
| | corn starch | salts | 500 - 550 | 1.62 |
| | potato starch | salts | 500 - 540 | 1.50 |
| | insoluble starch | salts | 480 - 520 | 1.55 |

### REFERENCES

1. Wong H.- C.; Koehler P.E., Production and isolation of antibiotic from *Monascus purpureus* and its relationship to pigment production, 1981, J.Food Sci., 46, 8, 589
2. JP 60 149 512, Make up containing natural colours, abstract 10374 in Chemical Abstracts, vol 104, 1986.
3. Martin St., Production of crystaline pigment from *Monascus* during fermentation. US Patent 4 927 760, 1989.
4. Shepherd D.M., Carels M., Red pigment production, US Patent 4 145 254, 1979.
5. Yueh M., Rashbaum S. A., Natural red colouring prepared from wheat and barley substrates, US Patent 4 418 080, 1983.
6. Grzybowski R., Stegman I., Stimulation of the biosynthesis of pigments by *Monascus purpureus* fungus, Przem. Spozyw., 1985, 39, 402 - 404. Abstract 194726s in Chemical Abstracts, vol. 106, 1967.
7 . Somogyi M.J. Notes on sugar determination. J. Biol. Chem. 1952, 195, 19.
8. Jacobs. S.. Meth. biochem. Anal., 13. 241, 1965.
9. King E.J., Biochem. J. 26, 292, 1932.
10. Lowry O.H., Rosenbrought N.J. Farel O.M., Randal V.J.. J. Biol. Chem. 1951, 193, 265.
11. Su Y.C.; Huang J.H.. Fermentative Production of anka pigments, 1980, Proc. Natl. Sci. Counc. ROC., 4, 2, 201.
12. Bau Y.S.; Wong H.C., Zink effect on growth, pigmentation and antibacterial activity of *Monascus purpureus,* 1977, Physiol. Plants, 46, 63.
13. Applied Biochemistry and Microbiology, 1973, ed publ. house Mir. Moscow, V. 7, 256.

## Claims

1. Method for obtaining of food colouring product from pigment synthesising fungi belonging to the genus *Monascus,* by cultivating them at 25 - 40°C at pH 4 - 7 wherein the submerged cultures are used with air supply 0.3 - 1 l/l/min, agitation 200 - 500 rpm, for 2 - 5 days on a medium containing 10 - 100 % whey ultrafiltration permeate or milk ultrafiltration permeate with/without 0.1 - 5 % carbon, 0.1 - 3 % nitrogen sources, 0.1 - 1 % minerals and drying the final fermentation broth.

2. Method according claim 1, characterized with that, as pigment producers, the following strains from fungi belonging to the genus *Monascus* are used : *Monascus purpureus* CBS 109.07, *Monascus purpureus* DSM 1604, *Monascus purpureus* DSM 1603, *Monascus ruber* DSM 62748.

3. Method according claim 1, characterized with that, as additional carbon source, sugars, aminosugars, sugar alcohols and alcohols (0,1 - 5 %) and natural sources of starch (0,5 - 3 %) are added.

4. Method according claim 1, characterized with that to the main culture medium additional nitrogen sources, ammonium, nitrates, yeast, malt, corn extract, etc. in concentration 0.1 - 3 %, are added.

5. Method according claim 1, characterized with that, as additional mineral salts, potassium, sodium, magnesium, sulphate and phosphate salts are added.

6. Method according claim 1, characterized with that the product obtained is concentrated by membrane filtration or vacuum evaporation and then is spray or freeze dried.

## Patentansprüche

1. Verfahren zur Gewinnung eines Produktes zum Färben von Lebensmitteln aus Pigment synthetisierenden Pilzen, die zu der Gattung *Monascus* gehören, bei dem diese bei 25 - 40°C bei pH 4 - 7 kultiviert werden, wobei die untergetauchten Kulturen mit einer Luftzufuhr von 0.3 - 1 l/l/min, einer Rührbewegung mit 200 - 500 U/min für 2 - 5 Tage auf einem Medium verwendet werden, das 10 - 100% Molke-Ultrafiltrationspermeat oder Milch-Ultrafiltrationspermeat mit/ohne 0.1 - 5% Kohlenstoff- und 0.1 - 3% Stickstoff-Quellen, 0.1 - 1% Mineralien enthält, und die End-Fermentationsbrühe getrocknet wird.

2. Verfahren nach Anspruch 1, **dadurch gekennzeichnet**, daß als Pigmentproduzenten die folgenden Stämme von zu der Gattung *Monascus* gehörenden Pilzen verwendet werden: *Monascus purpureus* CBS 109.07, *Monascus purpureus* DSM 1604, *Monascus purpureus* DSM 1603, *Monascus ruber* DSM 62748.

3. Verfahren nach Anspruch 1, **dadurch gekennzeichnet**, daß als zusätzliche Kohlenstoffquelle Zucker, Aminozucker, Zuckeralkohole und Alkohole (0,1 - 5%) und natürliche Quellen von Stärke (0,5 - 3%) hinzugefügt werden.

4. Verfahren nach Anspruch 1, **dadurch gekennzeichnet**, daß zu dem Haupt-Kulturmedium zusätzliche Stickstoffquellen, Ammonium, Nitrate, Hefe, Malz, Getreideextrakt, usw. in einer Konzentration von 0.1 - 3% hinzugefügt werden.

5. Verfahren nach Anspruch 1, **dadurch gekennzeichnet**, daß als zusätzliche Mineralsalze Kalium-, Natrium-, Magnesium-, Sulfat- und Phosphatsalze hinzugefügt werden.

6. Verfahren nach Anspruch 1, **dadurch gekennzeichnet**, daß das gewonnene Produkt durch Membranfiltration oder Vakuumverdampfung konzentriert wird und dann im Sprühverfahren getrocknet oder gefriergetrocknet wird.

## Revendications

1. Procédé d'obtention d'un produit colorant alimentaire à partir de champignons synthétisant un pigment appartenant au genre *Monascus,* par culture de ceux-ci à 25-40°C à pH 4-7,
dans lequel les cultures submergées sont utilisées avec une alimentation d'air de 0,3-1 l/l/mn, une agitation de 200-500 tours/mn, pendant 2-5 jours, dans un milieu contenant un perméat d'ultrafiltration à 10-100 % de petit lait ou un perméat d'ultrafiltration de lait avec ou sans sources de carbone (0,1-5%), d'azote (0,1-3%), et de minéraux (0,1-1%) et séchage du bouillon de fermentation final.

2. Procédé selon la revendication 1,
caractérisé en ce que, en tant que producteurs de pigments, on utilise les souches suivantes de champignons appartenant au genre *Monascus : Monascus purpureus* CBS 109.07, *Monascus purpureus* DSM 1604, *Monascus purpureus* DSM 1603, *Monascus ruber* DSM 62748.

3. Procédé selon la revendication 1,
caractérisé en ce qu'on ajoute, en tant que source de carbone additionnel, des sucres, des sucres aminés, des alcools de sucre, des alcools (0,1-5%) et des sources naturelles d'amidon (0,5-3%).

4. Procédé selon la revendication 1,
caractérisé en ce qu'on ajoute, au milieu de culture principal, des sources d'azote additionnel, de l'ammonium, des nitrates, des extraits de levure, de malt, de blé dans des concentrations de 0,1 à 3 %.

5. Procédé selon la revendication 1,
caractérisé en ce qu'on ajoute, en tant que sels minéraux additionnels, des sels de potassium, de sodium, de magnésium, des sulfates et des phosphates.

6. Procédé selon la revendication 1,
caractérisé en ce que le produit obtenu est concentré par filtration sur membrane ou évaporation sous vide et est ensuite séché par pulvérisation ou lyophilisation.
